# EUROPÄISCHE PATENTANMELDUNG

(11) **EP 0 913 177 A1**
(43) Veröffentlichungstag der Anmeldung: **06.05.1999**
(21) Anmeldenummer: 97119112.7
(22) Anmeldetag: 03.11.1997
(51) Int. Cl.: B01D 1/14, B01D 1/18, A61K 9/14, F26B 3/02

(54) **Verfahren zur Herstellung trockener, amorpher Produkte enthaltend biologisch aktive Materialien mittels Konvektionstrocknung, insbesondere Sprühtrocknung**

(71) Anmelder: Roche Diagnostics GmbH, 68298 Mannheim (DE)
(72) Erfinder: Gabel, Rolf-Dieter, Dr., 68723 Schwetzingen (DE); Mattern, Markus, Dr., 64646 Heppenheim (DE); Winter, Gerhard, Dr., 69221 Dossenheim (DE); Wirl, Alexander, Dipl.-Che.Ing., 67259 Heuchelheim (DE); Woog, Heinrich, Dr., 69514 Laudenbach (DE)

(57) **Zusammenfassung**

Die Erfindung betrifft ein schnelles und gut reproduzierbares Verfahren zur Herstellung von trocknen, amorphen Produkten, die neben biologisch aktivem, insbesondere therapeutisch aktivem Material, Substanzgemische zur Stabilisierung mittels Konvektionstrocknung enthalten. Gegenstand der Erfindung sind auch die mit diesem Verfahren erhaltenen amorphen oder teilamorphen, mikroskopisch homogenen Produkte, die als Pulver vorliegen und eine gleichmäßige geometrische, insbesondere sphärische, Gestalt aufweisen. Desweiteren betrifft die Erfindung die Verwendung von Substanzgemischen zur Stabilisierung von biologisch aktivem Material, insbesondere von Proteinen mittels Sprühtrocknung.

## Beschreibung

Die Erfindung betrifft ein schnelles und gut reproduzierbares Verfahren zur Herstellung von trocknen, amorphen Produkten, die neben biologisch aktivem, insbesondere therapeutisch aktivem Material, Substanzgemische zur Stabilisierung mittels Konvektionstrocknung enthalten Gegenstand der Erfindung sind auch die mit diesem Verfahren erhaltenen amorphen oder teilamorphen, mikroskopisch homogenen Produkte, die als Pulver vorliegen und eine gleichmäßige geometrische, insbesondere sphärische, Gestalt aufweisen. Desweiteren betrifft die Erfindung die Verwendung von Substanzgemischen zur Stabilisierung von biologisch aktivem Material, insbesondere von Proteinen mittels Sprühtrocknung.

Bekanntermaßen lassen sich Proteine, insbesondere Humanproteine, durch eine Vielzahl von Stoffen, bevorzugt durch Zucker oder Kombinationen von Zuckern und Aminosäuren, in festen Zubereitungen stabilisieren.

Verschiedene Verfahren und Formulierungen zur Herstellung von trockenen, biologisch oder therapeutisch aktiven Materialien sind beschrieben. Unter trockenen Materialien werden Stoffe und Stoffgemische verstanden, die eine Restfeuchte von höchstens 8% (g/g), bevorzugt höchstens 4% (g/g), besonders bevorzugt höchstens 2% besitzen. Gefriertrocknungsverfahren sind für die Herstellung solcher Formulierungen weit verbreitet [F. Franks, Cryo Lett 11, 93 - 110, (1990); M. J. Pikal, Biopharm. 3 (9), 26 - 30 (1990); M. Hora, Pharm. Research 8 (3), 285 - 291 (1992); F. Franks, Jap. J. Freezing Drying 38, 15 - 16, (1992)], weisen aber die spezifischen Nachteile der Gefriertrocknung auf Sie verbrauchen große Energiemengen, erfordern den Einsatz teilweise umweltschädlicher Kältemittel (Frigene), und sind zeitaufwendig, da es oft erforderlich ist, durch Sublimation größere Volumen von Eis zu entfernen. Für eine Vielzahl von Substanzen, insbesondere für Proteine, kann der für die Gefriertrocknung notwendige Schritt des Einfrierens destabilisierend sein. Daher ist dieses Verfahren für manche biologische Materialien gar nicht anwendbar.

Alternativen zur Gefriertrocknung bei der Herstellung trockener Proteinzubereitungen sind Verfahren, die durch Anwendung von Wärme und/oder Vakuum das Gut trocknen [F. Franks, R. M. H. Hatley: Stability and Stabilization of Enzymes; Eds. W. J. J. von den Teel, A. Harder, R. M. Butlaar, Elsevier Sci. Publ. 1993, pp. 45 - 54; B. Roser, Biopharm, 4(9), 47 - 53 (1991); J. F. Carpenter, J. H. Crowe, Cryobiol 25, 459 - 470 (1988)]. Hier sind beispielsweise Vakuumtrocknung mit oder ohne Anwendung von erhöhter Temperatur, Sprühtrocknungsverfahren in verschiedensten Modifikationen inkl. der kombinierten Anwendung von Vakuum und Sprühtechnik sowie Walzentrocknung u. a. Dünnschichttrockenverfahren zu nennen.

In J. F. Carpenter, J. H. Crowe, Biochemistry 28, 3916 - 3922 (1989); K. Tanaka T. Taluda, K. Miyajima, Chem. Pharm. Bull. 39 (5), 10 - 94 (1991), DE 35 20 228, EP 0 229 810, WO 91/18091, EP 0 383 569 bzw. US 5,290,765 werden Zubereitungen beschrieben, die Zucker oder zuckerartige Stoffe enthalten. Nach dem Stand der Technik ist jedoch die Herstellung von trockenen, kohlenhydrathaltigen Zubereitungen mittels Gefrier- oder Vakuumtrocknung, besonders von Zuckerzubereitungen, mit Nachteilen verbunden. Dazu gehören unter anderem, hoher Energieaufwand zur Trocknung auf eine akzeptable Restfeuchte, verlängerte Prozeßzeiten bei niedrigen Trocknungstemperaturen, Bildung von hochviskosen, wasserhaltigen Massen (sog. 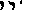 rubber") oder glasartigen Schmelzen, deren Glasübergangstemperaturen unterhalb der Raumtemperatur liegen. Die oben beschriebenen Nachteile beeinflussen die Stabilität von biologischen Materialien in solchen Zubereitungen signifikant.

Aus der oben zitierten Literatur geht auch hervor, daß zur Stabilisierung von Proteinen geeignete Zubereitungen glasartige, d.h. teil- oder vollamorphe Strukturen aufweisen sollen, deren Glasübergangstemperatur oberhalb der angestrebten Lagertemperatur liegt. Die Glasübergangstemperatur oder Glasumwandlungstemperatur (Tg) ist diejenige Temperatur, bei der ein amorpher oder teilkristalliner Festkörper aus dem Glaszustand in den flüssigen oder zäh-viskosen Zustand übergeht und umgekehrt. Dabei treten drastische Änderungen der Viskosität auf und damit verbunden des Diffusionskoeffizienten und der kinetischen Mobilität der biologischen Materialien. Physikalische Kenngrößen, wie Härte und Elastizitätsmodul ändern sich ebenso wie die thermodynamischen Zustandsgrößen Volumen, Enthalpie und Entropie. Die Glasübergangstemperatur einer beispielsweise zuckerhaltigen Masse und ihr Restwassergehalt sind physikalisch derart miteinander verknüpft, daß steigende Restwassermengen zu sinkenden Glasübergangstemperaturen führen und umgekehrt. Somit läßt sich aus der Messung der Glasübergangstemperatur, z.B. durch Differential Scanning Calorimetrie (DSC), ableiten, ob eine Zubereitung einen zur Stabilisierung geeigneten Restwassergehalt aufweist, bzw. wie oben ausgeführt, ein Trocknungsverfahren erfolgreich ist oder nicht. Neben der Bestimmung der Glasübergangstemperatur mittels DSC läßt sich das Vorhandensein amorpher Strukturen auch mittels Röntgenbeugungsuntersuchungen, optischer und elektronenmikroskopischer Betrachtungen belegen.

Wünschenswert war daher die Bereitstellung amorpher möglichst vollamorpher, Hilfsstoffgerüste für biologische oder pharmazeutisch aktive Materialien, damit die eingebetteten biologischen Materialien auch bei Raumtemperatur und über einen langen Zeitraum stabil gehalten werden können. Die Hilfstoffgerüste sollen eine geringe Restfeuchte enthalten, die gezielt einstellbar ist.

Der Erfindung lag also die Aufgabe zugrunde, ein schonendes, flexibles, gut reproduzierbares und schnelles Trocknungsverfahren für die Einbettung von biologischen Materialien, insbesondere von Humanproteinen, zu entwickeln und die für dieses Verfahren geeigneten Stabilisierungsmatrixes herauszufinden.

Die Aufgabe der Erfindung, ein effizientes Verfahren zur Herstellung amorpher Zubereitungen, die biologische Materialien enthalten, bereitzustellen, wird mittels Konvektionstrocknung, insbesondere mittels Spruhtrocknung, unter den in Anspruch 1 angegebenen Bedingungen und mit den in Anspruch 1 angegebenen Substanzgemischen gelöst. Gegenstand der Erfindung ist insbesondere ein Verfahren zur Herstellung von trockenen, amorphen Produkten, die neben biologischem Material, insbesondere therapeutisch aktivem Material, Substanzgemische zur Stabilisierung enthalten, das dadurch gekennzeichnet ist, daß eine Lösung oder Suspension aus biologischem Material und einem Substanzgemisch bestehend aus (a) einem Kohlenhydrat und mindestens einem Zwitterion mit polarem oder apolarem Rest oder deren Derivaten, und/oder (b) mindestens zwei Zwitterionen mit polaren oder apolaren Resten oder deren Derivaten, und/oder (c) mindestens von einem Zwitterion mit polarem oder apolarem Rest oder von mehreren Zwitterionen mit polaren oder apolaren Resten oder deren Derivaten, hergestellt wird und mittels Konvektionstrocknung unter Einstellung einer relativen Feuchte von < 70%, bevorzugt < 40 %, insbesondere < 20%, in der stationären Phase bei einer Zulufttemperatur von < 300 °C, bevorzugt < 200 °C getrocknet wird Diese Trocknungsart erweist sich als besonders vorteilhaft hinsichtlich der Stabilität der biologischen Materialien in den Formulierungen und gewährbeistet Ausbeuten > 90%.

Durch Sprühtrocknung mit den in Anspruch 1 angegebenen Merkmalen ist es möglich, schlecht trockenbare Kohlenhydrate, Aminosäuren oder deren Derivate so zu modifizieren, daß sie getrocknet werden können, oder Kohlenhydrate, Aminosäuren oder deren Derivate mit einer Substanz oder einem Substanzgemisch zu versetzen, die die Tg soweit erhöhen, daß eine Trocknung möglich wird und das entstehende Hilfsstoffgerüst teil- oder vollamorph ist und für die Einbettung von biologischen Materialien hervorragend geeignet.

Es wurde gefunden, daß bei Kohlenhydraten, insbesondere Zuckern wie z. B. Saccharose, Fructose, die nach dem Versprühen aus wäßriger oder organischer Lösung in ein Heißluftbett zwar amorphe Strukturen ausbilden, aber niedrige Glasübergangstemperaturen (<20°C) aufweisen und daher beim Trocknen in ökonomisch äußerst ungünstigen Ausbeuten anfallen und geringe Lagerstabilität besitzen, letzteres insbesondere hinsichtlich der Erhaltung amorpher Strukturen, durch Zugabe von Zwitterionen oder deren Derivaten die Tg soweit erhöht wird (>20°C), daß sie in guter Ausbeute getrocknet werden können und die teil- oder vollamorphe Struktur der Kohlenhydrate bzw. der Gesamtformulierung erhalten bleibt bzw. stabilisiert wird.

Weiterhin hat sich überraschend gezeigt, daß durch die Zugabe von Zwitterionen oder deren Derivaten erreicht werden kann, daß Kohlenhydrate, wie z.B. Mannit, nach dem Versprühen aus wäßriger oder organischer Lösung in ein Heißluftbett in einer teil- oder vollamorphen Struktur anfallen, wie sie für die Stabilisierung von biologischen Materialien erforderlich ist. Gleichzeitig werden hierbei gute Ausbeuten erzielt. Dieses Ergebnis ist insofern überraschend, da bei Durchführung dieses Verfahrens ohne den Zusatz von Zwitterionen keine amorphen Strukturen erhalten werden.

Andererseits wurde gefunden, daß auch Zwitterionen oder deren Derivate, die zwar nach dem Versprühen aus wäßriger oder organischer Lösung in einem Heißluftbett getrocknet werden können, aber dabei nicht in amorpher Form anfallen, durch Zugabe von Kohlenhydraten oder deren Derivaten in einer teil- oder vollamorphen Form anfallen.

Teil- oder vollamorphe Strukturen werden auch dann erhalten, wenn Zwitterionen oder deren Derivate, die zwar nach dem Versprühen aus wäßriger oder organischer Lösung in einem Heißluftbett getrocknet werden können, aber dabei nicht in einer teil- oder vollamorphen Form anfallen, durch geeignete Zumischungen von einem oder mehreren Zwitterionen in eine teil- oder vollamorphe Form übergeführt werden können. Zur Erzeugung der teil- oder vollamorphen Struktur ist es nicht erforderlich, nur Kombinationen aus Zwitterionen mit polaren Resten oder apolaren Resten zu wählen, sondern es können auch Kombinationen aus Zwitterionen mit nur polaren oder nur apolaren Resten eingesetzt werden.

Desweiteren zeigte sich, daß Zwitterionen oder deren Derivate oder Kombinationen davon, die zwar sprühgetrocknet werden können, aber nicht in amorpher Form anfallen, durch spezifische pH-Wert-Einstellung der Lösung in den amorphen Zustand übergeführt werden können.

Durch geeignete Kombination der aufgeführten Varianten ist der Fachmann in der Lage, die bereits erzielten Effekte noch signifikant zu erhöhen, Durch die Lehre des Patentes wird er in die Lage versetzt, ein Zwitterion mit polarem oder apolarem Rest so auszuwählen, daß das getrocknete Substanzgemisch einen erhöhten Glaspunkt und/oder eine teil- oder vollamorphe Struktur gegenüber einem Substanzgemisch ohne entsprechenden Zusatz aufweist.

Die mit dem erfindungsgemäßen Verfahren erhaltenen Produkte sind amorphe oder teilamorphe und mikroskopisch homogene Pulver in einem Korngrößenbereich von 0,0005 mm bis 1 mm, vorzugsweise von 0,001 mm bis 0,1 mm. Das erfindungsgemäße Verfahren erlaubt es, vorzugsweise sphärische Partikel in einem einstellbaren und überraschend gut reproduzierbaren Korngrößenbereich zu erhalten. Die erhaltenen Produkte besitzen eine Glasübergangstemperatur con ≥ 20°C, vorzugsweise ≥ 40°C und eine Restfeuchte < 8% (g/g), vorzugsweise < 4% (g/g). Die teil- oder vollamorphe Struktur werden ferner über Lagerzeiten von mindestens 12 Monaten beibehalten. Sie weisen im Vergleich zu Lyophilisaten eine mindestens um den Faktor 1,15 (15%) höhere scheinbare Dichte als Lyophilisate auf und haben gegenüber Produkten aus der Gefrier- oder Vakuumtrocknung bei gleicher Formulierung signifikant niedrigere kristalline Anteile.

Erfindungsgemäß werden die amorphen Produkte, die vorzugsweise Proteine als biologische Materialien enthalten, durch Konvektionstrocknung, insbesondere durch Sprühtrocknung oder Sprühgranulation, hergestellt, indem man von dem biologischen Material und dem Substanzgemisch eine Lösung oder Suspension herstellt und die Konvektionstrocknung bei einer Zulufttemperatur von <300°C, bevorzugt <200°C durchführt, wobei sich gezeigt hat, daß die relative Feuchte in der stationären Phase auf <70%, vorzugsweise <40%, eingestellt werden muß, um eine definierte Produktfeuchte von <4% zu erzielen. Das erfindungsgemäße Verfahren erlaubt es also, die gewünschte Restfeuchte in den Endprodukten gezielt einzustellen.

Erfindungsgemäß kann die Konvektionstrocknung mittels Fluidattrocknung, Förderluft- oder Flugtrocknung erfolgen. Erfindungsgemäß ganz besonders bevorzugt wird die Sprüh- oder Wirbelschichttrocknung angewandt.

Das zu trocknende Material wird bei der Sprühtrocknung in an sich bekannter Weise als Lösung oder Suspension am oberen Ende eines weiten, zylindrischen Behälters durch Düsen oder mittels einer schnell rotierenden Zerstäuberscheibe zu einem Tropfennebel versprüht. Der entstehende Tropfennebel wird mit heißer Luft oder einem Inertgas gemischt, welche um die Zerstäubungszone in den Trockner eingeleitet werden. Wird eine Lösung unter sonst gleichen Bedingungen mittels Zweistoffdüse oder Scheibe zerstäubt, ist die Korngrößenverteilung der Spruheinbettung der Scheibenzerstäubung gegenüber der Düsenzerstäubung zwar enger, aber in den gröberen Bereich verschoben.

Als Zerstäubungseinrichtungen kommen Drall-Druckdüsen, pneumatische Düsen (Zweistoff-/Dreistoffdüsen) oder Zentrifügalzerstäuber in Frage. Obwohl pneumatische Düsen zum Zerstauben pro kg Flüssigkeit den höchsten Energieaufwand erfordern, sind diese Düsen wegen ihrer Flexibilität besonders geeignet, z. B. zur Erzielung bestimmter Korngrößenbereiche oder bestimmter Teilchenformen. Das Verfahren kann auch mittels Anwendung einer Dreistoffdüsentechnik durchgeführt werden. Dadurch können zwei Flüssigkeiten gleichzeitig zerstäubt werden.

Dies ermöglicht zwei Varianten der Zerstäubung:

### Variante 1

Kurz vor der Zerstäubung werden die beiden getrennt zugeführten Flüssigkeiten gemischt und danach zerstäubt. Diese Variante findet dort bevorzugt Anwendung, wo die Mischungen aus zwei Komponenten in einer flüssigen Phase nur kurzfristig haltbar sind.

### Variante 2

Die beiden getrennt zugeführten Flüssigkeiten werden getrennt zerstäubt und vor der Düsenmündung gemischt. Diese Variante kann dort angewendet werden, wo zwei nicht mischbare Lösungen vorliegen oder die Stabilität der Komponenten nur im festem Zustand gewährleistet ist.

Diese speziellen Zerstäubungsarten waren für die Erfindung ebenso gut anwendbar wie die klassische Zweistoffzerstäubung.

Ein optimales erfindungsgemäßes Verfahren ist bei einer Zulufttemperatur im Bereich zwischen 100°C und 180°C gegeben. Da mit zunehmender Zulufttemperatur auch die Gefahr einer Zersetzung der Sprüheinbettung besteht, sind höhere Temperaturen ≥ 200°C normalerweise nicht ergiebig, können aber für spezielle Anwendungsfälle durchaus in Erwägung gezogen werden. Es ist überraschend, daß die Stabilität der biologischen Materialien in den Formulierungen sehr gut ist, obwohl die erfindungsgemäß zur Trocknung verwendete Heißluft vorzugsweise eine Temperatur von >100°C aufweist.

Durch das Versprühen der Lösung nach dem Trocknen fallen vorzugsweise sphärische Partikel in einem einstellbaren und reproduzierbaren Korngrößenbereich an. Gerade sphärische Formen mit günstigen Fließeigenschaften und einem bestimmten Korngrößenbereich sind besonders vorteilhaft für viele pharmazeutische Anwendungsformen.

Die Trocknungszeiten betragen mit dem erfindungsgemäßen Verfahren nicht mehr als eine Minute.

Es ist erfindungsgemäß auch möglich, Granulate herzustellen, indem die Lösung aus biologischem Material und Substanzgemisch auf einen Träger mit einem Korngrößenbereich von 0,010 mm bis 10 mm, bevorzugt 0,1 bis 1 mm, aufgesprüht wird.

Mit dem erfindungsgemäßen Verfahren ist es durch Verwendung der angeführten Substanzgemische also möglich, die Trocknung kohlenhydrathaltiger Massen entscheidend zu verbessern. Die erfindungsgemäßen Formulierungen enthalten als Hauptbestandteil ein Kohlenhydrat, bevorzugt Zucker, und ein oder mehrere Zwitterionen mit polaren oder unpolaren Resten oder deren Derivate, wobei die Glasübergangstemperatur des Zuckers durch diesen Zwitterionzusatz deutlich erhöht wird.

Formulierungen, bei denen der Hauptbestandteil Zwitterionen mit polaren oder unpolaren Resten oder deren Derivate sind, können durch Zusatz von Kohlenhydraten in stabile amorphe Formen übergeführt werden.

Alternativ zu den Kohlenhydraten können auch Zwitterionen mit polaren oder unpolaren Resten oder deren Derivate oder Gemische davon verwendet werden. Diese Formulierungen können sich aus mindestens 2 Zwitterionen oder deren Derivate mit polaren Resten, mindestens 2 Zwitterionen oder deren Derivate mit apolaren Resten oder aus mindestens einem Zwitterion mit polarem und apolarem Rest zusammensetzen.

Teil- oder vollamorphe und trockene Formulierungen können auch durch spezifische pH-Werteinstellung der Lösung von Zwitterionen mit polaren oder apolaren Resten oder deren Derivate, alleine oder in Kombination erreicht werden.

Erfindungsgemäß sind Substanzgemische aus mindestens zwei Substanzen ausgewählt aus Saccharose, L-Arginin, L-Phenylalanin, L-Aspartinsäure, L-Isoleucin und deren Derivate bevorzugt einsetzbar.

Neben den in den Beispielen angegebenen Zusammensetzungen erwiesen sich auch die folgenden Substanzgemische als Matrix zur Bildung erfindungsgemäßer Sprühprodukte als besonders geeignet:
Gemisch (Rezeptur) 1:
   Saccharose, L-Arginin und L-Phenylalanin,
Gemisch (Rezeptur) 2:
   L-Arginin, L-Aspartinsäure und L-Isoleucin,
Gemisch (Rezeptur) 3:
   L-Arginin und L-Phenylalanin,
Gemisch (Rezeptur) 4:
   L-Arginin, L-Phenylalanin und L-Aspartinsäure,

Gegenstand der Erfindung ist auch die Verwendung, der erfindungsgemäß hergestellten amorphen oder teilamorhen Produkte zur Herstellung diagnostischer oder therapeutischer Mittel, indem sie gegebenenfalls mit üblichen Hilfs- und Trägerstoffen weiterverarbeitet werden.

### Beispiel 1

Das Kohlenhydrat und die Aminosäure (AS) wurden in Wasser bei RT gelöst. Je nach verwendetem biologischen Material kann eine pH-Werteinstellung erforderlich sein. Anschließend wird diese Lösung sprühgetrocknet.

Aus folgender Tabelle ist ersichtlich, daß z. B. Saccharose oder Fructose wegen der niedrigen Glasübergangstemperatur nicht in ökonomisch guten Ausbeuten getrocknet werden können (1.1 und 1.5), wahrend die Lösungen mit AS-Zusatz nach dem Trocknen als feinteilige, trockene Pulver in guten Ausbeuten anfallen (1.2 - 1.4 und 1.6). Weiterhin ist aus der Tabelle ersichtlich, daß Kohlenhydrate wie z.B. Mannit, die üblicherweise nach dem Trocknen keine amorphen Strukturen ausbilden und in ökonomisch günstigen Ausbeuten anfallen (1.7), durch Zugabe von Zwitterionen oder deren Derivaten eine amorphe Struktur ausbilden und in guten Ausbeuten anfallen, wie sie für die Stabilisierung biologischer Materialien erforderlich sind (1.8).

### Kohlenhydrate mit Zusatz an Zwitterionen mit polaren/apolaren Resten

| **Beispiel** | **Kohlenhydrat mg/ml** | **Aminosäure mg/ml** | **Restwasser gehalt %** | **Glasübergangstemperatur °C Kristallstruktur Aggregatzustand** |
|---|---|---|---|---|
| | Saccharose | L-Arginin | | |
| (1.1) | 275 | 0 | 2,8 | <20°C röntgenamorph klebrige Masse |

| | Saccharose | L-Arginin | | |
|---|---|---|---|---|
| (1.2)* | 100 | 5 | 2,4 | ≥**20°C** röntgenamorph Pulver |

| | Saccharose | L-Phenylalanin | | |
|---|---|---|---|---|
| (1.3) | 100 | 5 | 2,8 | ≥**20°C** röntgenamorph Pulver |
| (1.4) | 100 | 20 | 2,5 | ≥**20°C** röntgenamorph Pulver |

| | Fructose | L-Phenylalanin | | |
|---|---|---|---|---|
| (1.5) | 275 | 0 | 2,3 | <20°C röntgenamorph klebrige Masse |
| (1.6) | 100 | 5 | 4,0 | ≥**20°C** röntgenamorph Pulver |

| | Mannit | L-Arginin/L- Phenylalanin | | |
|---|---|---|---|---|
| (1.7) | 55 | 0 | 0,4 | -kristallin Pulver |
| (1.8)* | 100 | 10/10 | 2,5 | ≥**20°C** röntgenamorph Pulver |

| | | | | |
|---|---|---|---|---|
| * pH-Wert Einstellung auf 7.3 ± 0.2 | | | | |

### Beispiel 2

Die Aminosäure und das Kohlenhydrat wurden in Wasser bei RT gelöst und sprühgetrocknet. Aus der folgenden Tabelle ist ersichtlich, daß die reine AS in kristalliner Kristallstruktur anfällt (2.1 und 2.4), bei Kohlenhydrat-Zusatz aber amorphe Strukturen erhalten werden (2.2, 2.3,2.5).

### Zwitterionen mit polaren/apolaren Resten unter Zusatz von Kohlenhydraten

| **Beispiel** | **Aminosäure mg/ml** | **Kohlenhydrat mg/ml** | **Restwassergehalt %** | **Glasübergangstemperatur °C Kristallstruktur Aggregatzustand** |
|---|---|---|---|---|
| | L-Arginin | Methylhydroxypropylcellulose 2910 | | |
| (2.1) | 55 | 0 | 10,2 | -kristallin Pulver |
| (2.2) | 20 | 4 | 7,2 | ≥20°C röntgenamorph Pulver |

| | L-Arginin | Saccharose | | |
|---|---|---|---|---|
| (2.3) | 20 | 4 | 6,4 | ≥20°C röntgenamorph Pulver |

| | L-Phenylalanin | Saccharose | | |
|---|---|---|---|---|
| (2.4) | 55 | 0 | 0,4 | -kristallin Pulver |
| (2.5) | 20 | 4 | 4,6 | ≥20°C röntgenamorph Pulver |

### Beispiel 3

Die Aminosäuren wurden in Wasser bei RT gelöst und sprühgetrocknet. Der Tabelle ist zu entnehmen, daß die reine AS in kristalliner Struktur anfällt (3.1-3.4), bei Zusatz einer zweiten AS aber amorphe Strukturen erhalten werden (3.5-3.8). In den Beispielen 3.9-3.11 sind die Tg-Werte von Vakuumtrocknungen aufgezeigt. Die Tg-Werte bei der Vakuumtrocknung liegen bei vergleichbaren Rezepturen deutlich unterhalb der Werte des erfindungsgemäßen Verfahrens.

### Zwitterionen mit polaren/apolaren Resten unter weiteren Zusatz von Zwitterionen mit polare/ apolaren Resten

| **Beispiel** | **Aminosäure mg/ml** | **Aminosäure mg/ml** | **Restwassergehalt %** | **Glasübergangstemperatur °C Kristallstruktur Aggregatzustand** |
|---|---|---|---|---|
| | L-Arginin | | | |
| (3.1) | 55 | 0 | 10,2 | -kristallin Pulver |

| | L-Isoleucin | | | |
|---|---|---|---|---|
| (3.2) | 21.7 | 0 | 0,4 | -kristallin Pulver |

| | L-Phenylalanin | | | |
|---|---|---|---|---|
| (3.3) | 21.7 | 0 | 0,4 | -kristallin Pulver |

| | L-Aspartinsäure | | | |
|---|---|---|---|---|
| (3.4) | 6,7 | 0 | 0,3 | -kristallin Pulver |

| | L-Phenylalanin | L-Isoleucin | | |
|---|---|---|---|---|
| (3.5) | 20 | 4 | 1,2 | ≥-20°C röntgenamorph Pulver |

| | L-Arginin | L-Phenylalanin | | |
|---|---|---|---|---|
| (3.6) | 20 | 4 | 5,7 | ≥20°C röntgenamorph Pulver |

| | L-Arginin | L-Aspartinsäure | | |
|---|---|---|---|---|
| (3.7) | 4 | 20 | 5,5 | ≥20°C röntgenamorph Pulver |

| | L-Arginin | L-Aspartinsaure/L- Isoleucin | | |
|---|---|---|---|---|
| (3.8) | 40 | 22 | 4,2 | ≥20°C röntgenamorph Pulver |
| | | 10 | | |

### Vaküumtrocknung

| Beispiel | Aminosäure mg/ml | Aminosäure mg/ml | Restwassergehalt % | Glasübergangstemperatur °C |
|---|---|---|---|---|
| | L-Arginin | L-Phenylalanin | | |
| 3.9 | 33,4 | 7,9 | 9,9 | 1,3 |
| 3.10 | 34,8 | 6,6 | 10,7 | 0,0 |
| 3.11 | 35,8 | 5,6 | 10,0 | 1,3 |

### Beispiel 4

Die Aminosäure wurde in Wasser bei RT gelöst und sprühgetrocknet. Aus folgender Tabelle ist ersichtlich, daß sich durch spezifische pH-Werteinstellung eine röntgenamorphe Struktur ausbildet (4.1 und 4.3), ansonsten nur eine kristalline Struktur erhalten wird (4.2 und 4.4.).

### Zwitterionen mit polaren/apolaren Resten

| **Beispiel** | **Aminosäure mg/ml** | **Aminosäure mg/ml** | **Restwassergehalt %** | **Glasübergangstemperatur °C Kristallstruktur Aggregatzustand** |
|---|---|---|---|---|
| | L-Arginin | | | |
| (4.1) | 55 pH 7,2 mit H₃PO₄ eingestellt | 0 | 5,1 | ≥20°C röntgenamorph Pulver |

| | L-Arginin | | | |
|---|---|---|---|---|
| (4.2) | 55 | 0 | 10,2 | -kristallin Pulver |

| | L-Aspartinsäure | | | |
|---|---|---|---|---|
| (4.3) | 8,3 pH 7,2 mit NaOH eingestellt | 0 | 0,5 | ≥20°C röntgenamorph Pulver |

| | L-Aspartinsäure | | | |
|---|---|---|---|---|
| (4.4) | 6,7 | 0 | 0,3 | -kristallin Pulver |

### Beispiel 5

Das Kohlenhydrat, die AS und sonstige Hilfsstoffe wurden in Wasser bei RT gelöst, der pH-Wert auf 73 ± 0.2 eingestellt und sprühgetrocknet.

Folgende Tabelle zeigt optimierte Verum-Rezepturen nach dem erfindungsgemäßen Verfahren (5.2-5.6). Als Vergleich dient eine Placebo-Rezeptur (5.1). Alle Rezepturen weisen nach der Trocknung keine Zersetzungsprodukte auf wobei bei den Rezepturen 5.2-5.6 hochmolekulare (HMW-) Aggregate und EPO-Dimere höchstens bis 0,2% detektierbar waren. Dieser Wert wird auch nach 3-monatiger Lagerung nicht überschritten (aufgezeigt am Beispiel 5.3).

Dagegen weist eine nicht erfindungsgemäße Rezeptur direkt nach dem Trocknen schon einen sehr hohen Gehalt an hochmolekularen (HMW-) Aggregaten und EPO-Dimeren auf (5.7).

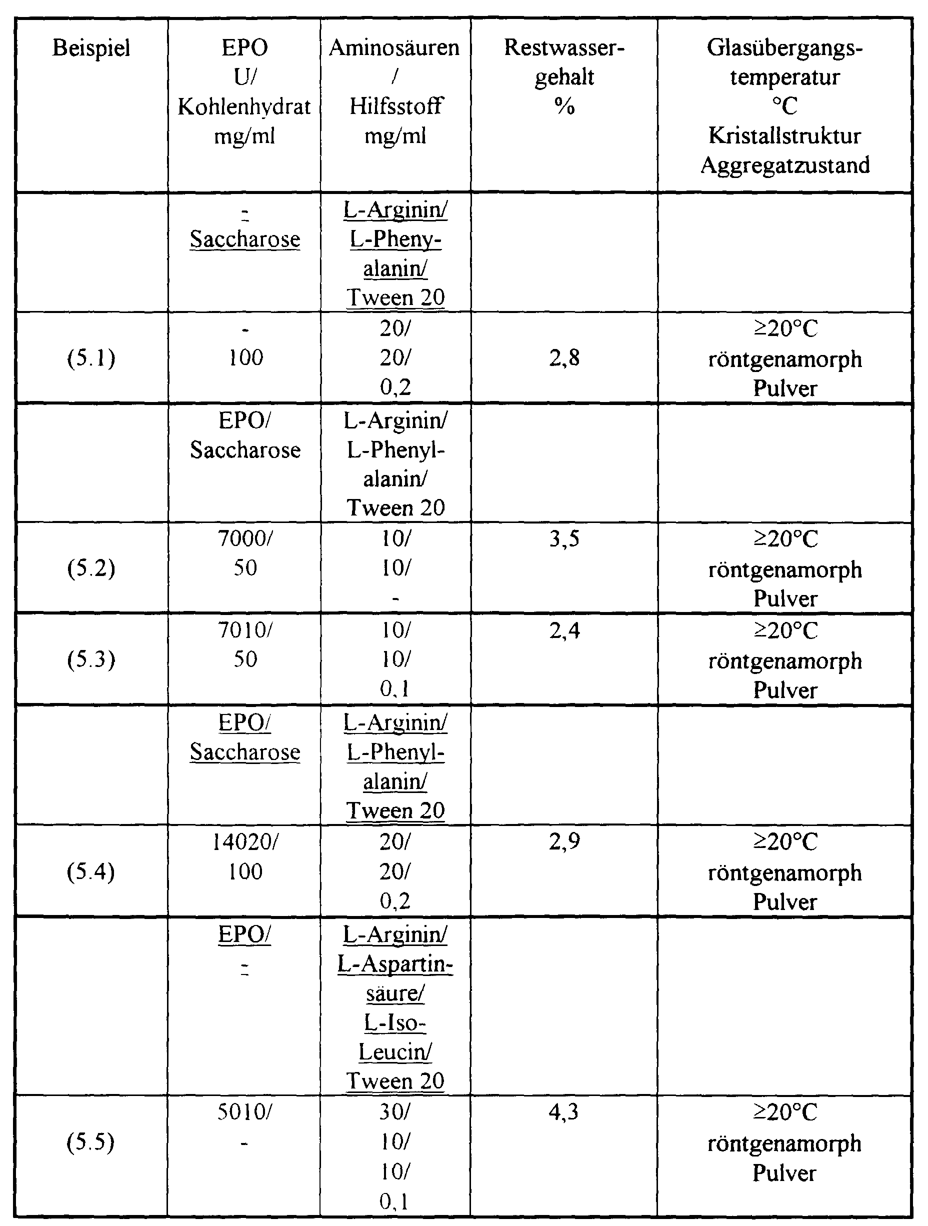

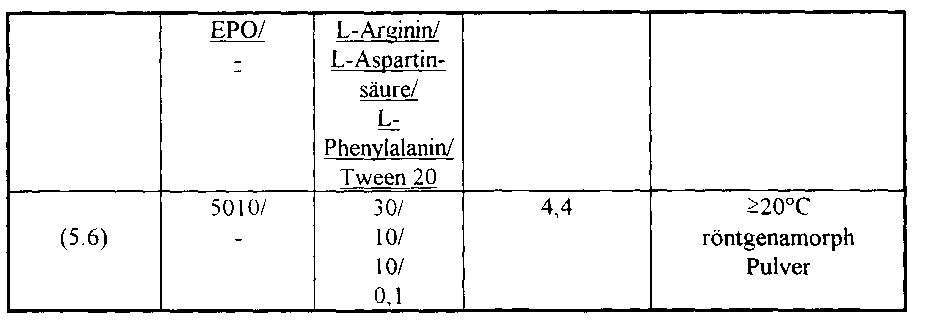

### Lagerstabilität von Beispiel 5.3 bei KS, RT und 40°C über 3 Monate

| | HMW-Aggregate | | | EPO-Dimere | | |
|---|---|---|---|---|---|---|
| | KS (4-6°C) | RT (20-22°C) | 40°C | KS (4-6°C) | RT (20-22°C) | 40°C |
| 5.3 | | | | | | |
| Anfangswert | | < 0,1% | | | < 0,1% | |
| nach 2 Monaten Belastung | < 0,1% | < 0,1% | < 0,1% | < 0,1% | < 0,1% | < 0,1% |
| nach 3 Monaten Belastung | < 0,1% | < 0,1% | < 0,1% | < 0,1% | < 0,1% | 0,1% |

Gehalt an HMW-Aggregaten und EPO-Dimeren in einer nicht erfindungsgemäßen Rezeptur direkt nach dem Trocknen:

| Beispiel | EPO U/Kohlenhydrat mg/ml | Restwasser gehalt % | HMW-Aggregate % | EPO-Dimere % |
|---|---|---|---|---|
| | EPO/Lactose | | | |
| (5.7) | 7010/50 | 3,4 | 9 | 2 |

### Beispiel 6

Die nachfolgenden Ausführungsbeispiele 6 bis 12 verdeutlichen den Einfluß der gewählten Bedingungen des erfindungsgemäßen Verfahrens auf die Endprodukte. Es wurden die folgenden Rezepturen 1 bis 4 untersucht:
Gemisch (Rezeptur) 1:
   Saccharose, L-Arginin und L-Phenylalanin, (5:1:1)
Gemisch (Rezeptur) 2:
   L-Arginin, L-Aspartinsäure und L-Isoleucin, (3:1:1)
Gemisch (Rezeptur) 3:
   L-Arginin und L-Phenylalanin (1:1)
Gemisch (Rezeptur) 4:
   L-Arginin, L-Phenylalanin und L-Aspartinsäure, (3:1:1)

### Sprühtrocknung der Substanzgemische 1 bis 4:

| **Einsatzstoffe** | **Zusammensetzung der Lösung** | | | |
|---|---|---|---|---|
| Gemisch | 1 | 2 | 3 | 4 |
| Saccharose | 50 mg/ml | | | |
| L-Arginin | 10 mg/ml | 30 mg/ml | 20 mg/ml | 30 mg/ml |
| L-Phenylalanin | 10 mg/ml | | 20mg/ml | 10 mg/ml |
| L-Aspartinsäure | | 10 mg/ml | | 10 mg/ml |
| L-Isoleucin | | 10 mg/ml | | |

### Beispiel 7

### Sprühtrocknung bei unterschiedlichen Zulufttemperaturen

Die vier Gemische wurden bei drei unterschiedlichen Zulufttemperaturen sprühgetrocknet, nämlich bei 100 °C, 140 °C und 180 °C. Ein sehr wichtiger Parameter bei der Sprühtrocknung ist die rel. Feuchte in der stationären Phase der Sprühtrocknung. Als stationäre Phase wird der Trocknungsabschnitt angesehen, wo der Trocknungsvorgang der versprühten Teilchen abgeschlossen ist und die maximale Temperaturbelastung der getrockneten Sprühteilchen erreicht wird. Sprühteilchen erreicht wird. Die relative Feuchte in der stationären Phase bestimmt die Produktfeuchte nach der Trocknung. Der hier zu wählende Wert der relativen Feuchte ist abhängig von der Zusammensetzung der Formulierung. Erfindungsgemäß wurde die relative Feuchte in der stationären Phase bei den vier Gemischen mit kleiner 40% (hier konkret: ca. 10%) sehr niedrig gewählt. Darnit wurde festgestellt, ob eine Sprühtrocknung der Gemische 1 - 4 überhaupt noch möglich Ist. Die Sprühtrocknung der vier Gemische konnte bei den oben genannten Bedingungen gut durchgeführt werden. In allen Fällen wurden feinpulvrige Sprüheinbettungen (SE) in guter Ausbeute (>90%) erhalten. Der Turmkonus und die Rohrleitungen waren nicht belegt, die Produkte konnten trotz der geringen Staub-/Luftverhältnisse (<<50 g/Nm³) gut ausgetragen werden.

### Relative Feuchte 10%

| Zulufttemperatur | Rel. Feuchte, stationäre Phase | Wasserverdampfungsleistung | Feststoff (aus 7%-iger Lösung) |
|---|---|---|---|
| °C | % | ca. g/h | ca. g/h |
| 60 | 10 | 100 | 7,5 |
| 100 | 10 | 600 | 45,2 |
| 140 | 10 | 1000 | 75,3 |
| 180 | 10 | 1400 | 105,4 |

### Beispiel 8

### Prüfung der Sprüheinbettungsprodukte (SE)

### a) pH-Wert, Dichte, Wassergehalt, Osmolalität

| Gemisch | Zulufttemperatur | Wassergehalt SE | pH-Wert Ausgangslsg./SE | Dichte Ausgangslsg./SE | Osmolalität Ausgangslsg./SE |
|---|---|---|---|---|---|
| | °C | % | | g/cm³ | mOsm/kg |
| Gemisch 1 | 100 | 3,2 | 7,4/7,44 | 1,025/1,023 | 270/272 |
| Gemisch 1 | 140 | 3,0 | 7,4/7,44 | 1,025/1,022 | 270/270 |
| Gemisch 1 | 180 | 3,0 | 7,4/7,43 | 1,025/1,020 | 270/273 |
| Gemisch 2 | 100 | 3,9 | 7,4/7,44 | 1,017/1,021 | 292/270 |
| Gemisch 2 | 180 | 2,6 | 7,4/7,44 | 1,017/1,022 | 292/279 |
| Gemisch 3 | 100 | 2,4 | 7,4/7,43 | 1,013/1,010 | 216/213 |
| Gemisch 3 | 180 | 2,7 | 7,4/7,43 | 1,013/1,011 | 216/212 |
| Gemisch 4 | 100 | 3,6 | 7,4/7,38 | 1,018/1,023 | 274/254 |
| Gemisch 4 | 180 | 2,7 | 7,4/7,37 | 1,018/1,023 | 274/254 |

Die in Wasser aufgelösten Sprüheinbettungen ergaben beim pH-Wert, der Dichte und der Osmolalität identische Werte wie bei der Ausgangslösung. Der Wassergehalt der Sprüheinbettungen erniedrigte sich geringfügig mit steigender Zulufttemperatur, obwohl die rel. Feuchte in der stationären Phase konstant gehalten wurde. Insbesondere Gemisch 1 weist bei allen Zulufttemperaturen einen fast konstanten Wassergehalt auf.

### b) Kristallstruktur

Unabhängig von den Zulufttemperaturen liegen alle Sprüheinbettungen im Vergleich zu den Ausgangsmischungen röntgenamorph vor.

### c) Elektronenmikroskopische Aufnahmen

Die elektronenmikroskopischen Aufnahmen an den Sprüheinbettungen von Gemisch 1 zeigen, daß die SE-Teilchen als fast ideale Vollkugeln vorliegen , wobei mit steigender Zulufttemperatur die Oberfläche von einer golfballartigen Struktur in ein glattes Aussehen übergeht. Zum Nachweis, daß Vollkugeln vorliegen, wurden die Sprüheinbettungen gemahlen. Anhand der Bruchstücke kann eindeutig auf die Vollkugelform geschlossen werden. Die Zerstaubungsart, Düsen- oder Scheibenzerstäubung, hat keinerlei Einfluß auf die Gestalt der SE-Teilchen.

### d) Korngrößenverteilung

Anhand von Gemisch 1 wurde nachgewiesen, daß die Korngrößenverteilung bei unterschiedlichen Sprühraten annähernd konstant bleibt.

| Zulufttemperatur | Rel. Feucht, stationäre Phase | Sprührate | Korngröße d10/d50/d90 | Korngröße RRSB-d' |
|---|---|---|---|---|
| °C | % | g/h | µm | µm |
| 100 | 10 | 640,0 | 0,8/3,7/9,7 | 5,7 |
| 140 | 10 | 1035,0 | 0,6/5,3/12,6 | 6,6 |
| 180 | 10 | 1455,0 | 0,7/4,0/11,9 | 6,3 |

Außerdem wurde festgestellt, daß man fast identische Komgrößenverteilungen bei gleicher Zulufttemperatur und annähernd gleicher Sprührate bekommt.

| Zulufttemperatur | Rel. Feucht, stationäre Phase | Sprührate | Korngröße d10/d50/d90 | Korngröße RRSB-d' |
|---|---|---|---|---|
| °C | % | g/h | µm | µm |
| 100 | 10 | 566,0 | 0,7/4,0/10,5 | 5,8 |
| 100 | 10 | 640,0 | 0,8/3,7/9,7 | 5,7 |
| 140 | 10 | 1067,5 | 0,8/4,0/11,6 | 6,2 |
| 140 | 10 | 1035,0 | 0,6/5,3/12,6 | 6,6 |

### Beispiel 9

### Messung der Glasübergangstemperatur (Tg) durch Differential Scanning Calorimetrie (DSC)

Zur Ermittlung der Glasübergänge der getrockneten Proben, sowie der Kristallisations und Schmelzpeaks wurde ein DSC 7 Gerät der Fa. Perkin-Elmer (Überlingen) mit CCA 7 Tieftemperaturregelung mit flüssigem Stickstoff (Messer, Griesheim) und einem TAC 7/DX Signalumwandler verwendet. Die zwischen 5 und 20 mg schweren Proben wurden in zuvor mittels einer Autobalance AD4 Mikrowaage (Perkin-Elmer) tarierte Aluminiumtiegel (Perkin-Elmer) eingewogen. Dann wurden die Tiegel mit einem Deckel (Cover, Perkin-Elmer) mit einer Universalverschlußpresse (Perkin-Elmer) dicht verschlossen und in die stickstoffgeflutete Meßzelle gebracht und mit mit einer Aufheizrate von 10°C/min gemessen.

Es wurden die Tg mittels DSC von Gemisch 1 bestimmt. Die jeweiligen Tg lagen bei einem Wassergehalt < 4 % vorteilhafterweise weit über der Raumtemperatur, wodurch die Gemische zur Stabilisierung von biologischem Material, insbesondere von Humanproteinen gut geeignet sind.

| Zulufttemperatur | SE-Wassergehalt | Tg |
|---|---|---|
| °C | % | °C |
| 100 | 3,2 | 57,6 |
| 140 | 3,0 | 58,8 |
| 100 | 3,8 | 57,0 |
| 100 | 7,5 | 28,2 |

### Beispiel 10

### Unterschiedliche rel. Feuchte im stationären Zustand

An Gemisch 1 wird aufgezeigt, inwieweit die rel. Feuchte der stationären Phase bei sonst konstanten Bedingungen die Produktfeuchte und damit die Tg beeinflußt.

| Zulufttemperatur | Rel. Feuchte, stationäre Phase | Wassergehalt | Tg |
|---|---|---|---|
| °C | % | % | °C |
| 140 | 4 | 1,5 | 67,7 |
| 140 | 10 | 3,0 | 58,8 |
| 140 | 25 | 5,9 | 31,7 |
| 140 | 33 | 6,5 | 29,1 |
| 140 | 36 | 7,6 | 28,2 |

### Beispiel 11

### Einfluß der Ausgangskonzentration der Lösung auf die Sprüheinbettung

Eine Erhöhung der Ausgangskonzentration der Lösung ist grundsätzlich bis zu der von den Hilfsstoffen vorgegebenen Löslichkeitsgrenzen möglich und führt zu fast gleichen physikalischen Eigenschaften der Sprüheinbettungen wie aus einer 7%-igen Lösung. Dies wird anhand von Gemisch 1 mit folgender Tabelle verdeutlicht.

| Zulufttemperatur | Konzentration der Lösung | Tg/Wassergehalt/ pH | Korngröße d10/d50/d90 | Korngröße RRSB- d' |
|---|---|---|---|---|
| °C | % | °C/% | µm | µm |
| 100 | 15 | 57,3/3,6/7,5 | 0,8/4,6/12,9 | 6,7 |
| 100 | 15 | 52,8/3,4/7,44 | 0,7/4,6/10,8 | 6,0 |
| 100 | 7 | 57,6/3,2/7,38 | 0,8/3,7/9,7 | 5,7 |

### Beispiel 12

### a) Überpüfung verschiedener Zerstäubungsaggregate (Zweistoffdüse, Zerstäuberscheibe) unter Verwendung von Gemisch 1

Die Verwendung von verschiedenen Zerstäubungsaggregaten hat Auswirkungen auf die Komgrößenverteilungen der Sprüheinbettungen.

| Zulufttemperatur | Zerstäubungsaggregat | Zerstäubungsdruck bzw. Drehzahl | Korngröße d10/d50/d90 | Korngröße RRSB-d' | Steigung der Geraden im RRSB-Netz |
|---|---|---|---|---|---|
| °C | µm | bar/Upm | µm | µm | 1 |
| 100 | Zweistoffdüse | 3 bar | 0,8/3,7/9,7 | 5,7 | 1,018 |
| 140 | Zweistoffdüse | 3 bar | 0,6/5,3/12,6 | 6,6 | 1,052 |
| 180 | Zweistoffdüse | 3 bar | 0,7/4,0/11,9 | 6,3 | 0,994 |
| 100 | Scheibe | max. | 1,7/11,9/23,3 | 15,3 | 1,238 |

Für die pulmonale Anwendungen der SE-Produkte, die Teilchengrößen <10 µm erfordern, kommt eine Scheibenzerstäubung nicht in Frage. Zudem ist die Flexibilität der Zweistoffdüse zur Verschiebung von Korngrößenbereichen gegenüber einer Scheibe wesentlich höher Nachteil ist. daß bei der Zweistoff-Zerstäubung im Sterilbetrieb das Zerstäubungsmedium steril filtriert werden muß. Die sonstigen physikalischen Parameter der SE-Produkte sind unabhängig von der Zerstäubungsart, Aussehen mit eingeschlossen. Als Zerstäubungsmedium können die bekannten Medien wie Preßluft oder Inertgase, wie z.B. Edelgase (Neon, Argon, etc.) oder Kohlendioxid, eingesetzt werden.

### b) Unterschiedliche Düsenkombinationen

Mit den speziellen Düsenkombinationen (Dreistoffdüsen) werden zwei Flüssigkeiten gleichzeitig zugeführt und zerstäubt.

Mit beiden Varianten der Dreistoffdüse wurden Sprüheinbettungen erhalten, deren physikalische Parameter mit denen aus der Zweistoffzerstäubung erhaltenen Sprüheinbettungen übereinstimmen. Selbst die Tg und die röntgenamorphe Form waren identisch.

### Beispiel 13

### Reproduzierbarkeit bei gleichen/unterschiedlichen Ansatzgrößen

Die Reproduzierbarkeit des Verfahrens bei gleichen oder unterschiedlichen Ansatzgrößen ist gegeben und wird am Beispiel von Gemisch 1 gezeigt. In allen Fällen werden SE-Produkte erhalten bei denen die physikalischen Parameter der Sprüheinbettungen innerhalb sehr enger Grenzen zu liegen kommen. Somit können die Ergebnisse der Versuchsansätze direkt auf größere Ansätze übertragen werden und es steht damit ein produktionsreifes Verfahren zur Verfügung.

| Zulufttemperatur | Ansatzgröße | Kristallstruktur | Korngröße d10/d50/d90 | Korngröße RRSB- d' | DSC Tg/Wassergehalt |
|---|---|---|---|---|---|
| °C | St. | | µm | µm | °C/% |
| 100 | 1000 | röntgenamorph | 0,8/3,7/9,7 | 5,7 | 57,6/3,2 |
| 140 | 1000 | röntgenamorph | 0,6/5,3/12,6 | 6,6 | 58,8/3,0 |
| 180 | 1000 | röntgenamorph | 0,7/4,0/11,9 | 6,3 | 57,0/3,0 |
| 140 | 2000 | röntgenamorph | 0,8/4,0/11,6 | 6,2 | 58,8/2,8 |
| 100 | 300 | röntgenamorph | 0,7/4,0/10,5 | 5,8 | 57,0/3,2 |

## Patentansprüche

1. Verfahren zur Herstellung von trockenen, amorphen Produkten, die neben biologisch aktivem, insbesondere therapeutisch aktivem Material Substanzgemische zur Stabilisierung enthalten, dadurch gekennzeichnet, daß eine Lösung oder Suspension aus biologischem Material und einem Substanzgemisch bestehend aus
(a) einem Kohlenhydrat und mindestens einem Zwitterion mit polarem oder apolarem Rest oder deren Derivaten, und/oder
(b) mindestens zwei Zwitterionen mit polaren oder apolaren Resten oder deren Derivaten, und/oder
(c) mindestens von einem Zwitterion mit polarem oder apolarem Rest oder von mehreren Zwitterionen mit polaren oder apolaren Resten oder deren Derivaten,
hergestellt wird und mittels Konvektionstrocknung unter Einstellung einer relativen Feuchte von < 70% in der stationären Phase bei einer Zulufttemperatur von < 300 °C getrocknet wird.

2. Verfahren nach Anspruch 1, dadurch gekennzeichnet, daß als Zwitterionen mit polaren oder apolaren Resten Aminocarbonsäuren eingesetzt werden.

3. Verfahren nach Anspruch 1 oder 2, dadurch gekennzeichnet, daß Substanzgemische der Gruppe (a) eingesetzt werden, die Mono-, Oligo-, Polysaccharide, Arginin, Aspartinsäure, Citrullin, Glutaminsäure, Histidin, Lysin, Acetylphenylalaninethylester, Alanin, Cystein, Glycin, Isoleucin, Leucin, Methionin, Phenylalanin, Tryptophan, Valin und/oder deren Derivate umfaßt.

4. Verfahren nach Anspruch 1 oder 2, dadurch gekennzeichnet, daß Substanzgemische der Gruppe (b) eingesetzt werden, die Arginin, Aspartinsäure, Citrullin, Glutaminsäure, Histidin, Lysin, Acetylphenylalaninethylester, Alanin, Cystein, Glycin, Isoleucin, Leucin, Methionin, Phenylalanin, Tryptophan, Valin und/oder deren Derivate umfaßt.

5. Verfahren nach Anspruch 1 oder 2, dadurch gekennzeichnet, daß Substanzgemische der Gruppe (ci) eingesetzt werden, die Salze von Arginin, Aspartinsäure, Citrullin, Glutaminsäure, Histidin. Lysin, Acetylphenylalaninethylester, Alanin, Cystein, Glycin, Isoleucin, Leucin, Methionin, Phenylalanin, Tryptophan, Valin und/oder deren Derivate umfaßt.

6. Verfahren nach einem der Ansprüche 1 bis 5, dadurch gekennzeichnet, daß als biologisches Material eine oder mehrere Substanzen der Gruppen Proteine, Peptide, Glycoproteine, Lipoproteine, Enzyme, Coenzyme, Antikörper, Antikörperfragmente, Virusbestandteile, Zellen und Zellbestandteile, Vaccine, DNA, RNA, biologische Therapeutica und Diagnostica oder deren Derivate eingesetzt werden.

7. Verfahren nach einem der Ansprüche 1 bis 6, dadurch gekennzeichnet, daß der Lösung übliche Hilfsstoffe aus den Gruppen Puffer, Tenside, Antioxidantien, Isotonisierungsmittel, Konservierungsmittel zugesetzt werden.

8. Verfahren nach einem der Ansprüche 1 bis 7, dadurch gekennzeichnet, daß die Trocknung mittels Fluidattrocknung, Förderluft- oder Flugtrocknung erfolgt.

9. Verfahren nach einem der Ansprüche 1 bis 7, dadurch gekennzeichnet, daß die Trocknung mittels Sprüh- oder Wirbelschichttrocknung erfolgt.

10. Amorphe oder teilamorphe, mikroskopisch homogene Produkte, enthaltend ein biologisch aktives Material und ein Substanzgemisch zur Stabilisierung, mit einer Glasüberganstemperatur ≥ 20°C und einer Restfeuchte < 8% (g/g) erhalten gemäß der Ansprüche 1 bis 9.

11. Amorphe oder teilamorphe Produkte gemäß Anspruch 10, dadurch gekennzeichnet, daß sie eine Glasübergangstemperatur ≥ 40 °C und eine Restfeuchte < 4% (g/g) aufweisen.

12. Amorphe oder teilamorphe Produkte gemäß Anspruch 10 oder 11, dadurch gekennzeichnet, daß sie als Pulver in einem Korngrößenbereich von 0,0005 mm bis 1 mm, vorzugsweise 0,001 bis 0,1 mm. vorliegen.

13. Verwendung von amorphen oder teilamorphen Produkten der Ansprüche 10 bis 12 zur Herstellung von diagnostischen oder therapeutischen Mitteln.

14. Verwendung von Substanzgemischen bestehend aus
(a) einem Kohlenhydrat und mindestens einem Zwitterion mit polarem oder apolarem Rest oder deren Derivaten, und/oder
(b) mindestens zwei Zwitterionen mit polaren oder apolaren Resten oder deren Derivaten, und/oder
(c) mindestens einem Salz von einem Zwitterion mit polarem oder apolarem Rest oder mehreren Salzen von mehreren Zwitterionen mit polaren oder apolaren Resten oder deren Derivaten.
zur Stabilisierung von biologisch aktivem, insbesondere therapeutisch aktivem Material mittels Sprühtrocknung unter Einstellung einer relativen Feuchte von < 70% in der stationären Phase bei einer Zulufttemperatur von < 300 °C.
